# EUROPEAN PATENT SPECIFICATION

(11) **EP 1 633 327 B1**
(45) Date of publication and mention of the grant of the patent: **11.08.2010**
(21) Application number: 04754323.6
(22) Date of filing: 04.06.2004
(51) Int. Cl.: A61K 9/127, A61K 49/00

(54) **METHOD FOR IMPROVING STABILITY AND SHELF-LIFE OF LIPOSOME COMPLEXES**
VERFAHREN ZUR VERBESSERUNG DER STABILITÄT UND DER HALTBARKEIT VON LIPOSOM-KOMPLEXEN
METHODE D'AUGMENTATION DE LA STABILITE ET DE LA DUREE DE CONSERVATION DE COMPLEXES COMPRENANT UN LIPOSOME

(30) Priority: 04.06.2003 US 475500 P
(43) Date of publication of application: 15.03.2006
(73) Proprietor: GEORGETOWN UNIVERSITY, Washington, D.C. 20057 (US)
(72) Inventor: CHANG, Esther, H., Potomac, MA 20854 (US); PIROLLO, Kathleen F., Rockville, MA 20850 (US)
(74) Representative: Dzieglewska, Hanna Eva
(86) International application number: PCT/US2004/017695
(87) International publication number: WO 2005/000271

(56) References cited:
- US-A- 4 857 319
- US-A- 5 612 057
- US-A- 5 753 262
- US-A- 5 811 406
- US-B1- 6 372 250

## Description

This application claims priority from U.S. provisional application number 60/475,500, filed June 4, 2003 incorporated herein by reference in its entirety.

### Field of the Invention

This invention relates to a method of preparing a stable complex comprising a ligand and a cationic liposome encapsulating a therapeutic or diagnostic agent.

### Background of the Invention

Cationic liposomes are composed of positively charged lipid bilayers and can be complexed to negatively charged, naked DNA by simple mixing of lipids and DNA such that the resulting complex has a net positive charge. The complex can be bound to and taken up by cells in culture with moderately good transfection efficiency. Cationic liposomes have been proven to be safe and efficient for in vivo gene delivery.

Liposomes can be used to target tumor cells by modifying the liposomes-so that they selectively deliver their payload to tumor cells. Surface molecules can be used to target liposomes to tumor cells because the type and/or number of molecules that decorate the exterior of tumor cells differ from those on normal cells. For example, if a liposome has a folate or transferrin (Tf) molecule on its surface, it will home to cancer cells that have levels of the folate or transferrin receptor which are higher than those on normal cells.

In addition to the use of ligands that are recognized by receptors on tumor cells, specific antibodies also can be attached to the liposome surface, enabling them to be directed to specific tumor surface antigens (including, but not limited to, receptors). These "immunoliposomes" can deliver therapeutic drugs to a specific cell population. It has been found, for example, that anti-HER-2 monoclonal antibody (Mab) Fab fragments conjugated to liposomes could bind specifically to a breast cancer cell line, S-BR-3, that over-expresses HER-2 (Park, J.W., et al. PNAS 92:1327-1331 (1995)). The immunoliposomes were found to be internalized efficiently, and the anchoring of anti-HER-2 Fab fragments enhanced their inhibitory effects. The combination of cationic liposome-gene transfer and immunoliposome techniques appears to be a promising system for targeted gene therapy.

A ligand-targeted liposomal delivery system for DNA gene therapy possessing selective tumor targeting and high transfection efficiency has been described in the art. Xu, L., et al. Human Gene Therapy 8:467-475 (1997); Xu, L., et al., Human Gene Therapy 10:2941-2952 (1999); and Xu, L., et al., Tumor Targeting 4:92-104 (1999). This system has been improved through use of an anti-transferrin receptor single chain (TfRscFv) antibody fragment as the targeting ligand in the complex (Xu, L., et al. Molecule Medicine 7:723-734 (2001); Xu, L., et al. Molecular Cancer Therapeutics 1:337-346 (2002)). The TfRscFv is formed by connecting the component VH and VL variable domains from the light and heavy chains, respectively, with an appropriately designed linker peptide. The linker bridges the C-terminus of the first variable region and N-terminus of the second, ordered as either VH-linker-VL or VL-linker-VH. The binding site of an scFv can replicate both the affinity and specificity of its parent antibody bonding site.

Conventional treatments for cancer involve chemotherapy and/or radiation treatments. Incorporating into these conventional cancer therapies a new component which results in sensitization of tumors to the chemotherapy or radiation therapy would have great clinical relevance, lowering the effective doses of both types of anti-cancer modalities and correspondingly lessening the severe side effects often associated with these treatments.

Initial studies with liposome complexes as described above have shown that the complexes are efficient in delivering diagnostic or therapeutic agents to the target cells of interest. It is impractical to administer the complexes to a patient immediately upon their preparation. It would be desirable to provide targeted liposome complexes that upon lyophilization and storage at 2-8°C, -20°C or - 80°C remain stable for at least six months and can be reconstituted without a significant loss of activity.

Previous reports have indicated that a two-component complex (lipid and DNA but without targeting ligand or proteins) could be lyophilized in the presence of mono- or di-saccharides and still maintain their biological activity and a particle size appropriate for gene therapy (Li, B., et al., Journal of Pharmaceutical Sciences 89:355-364 (2000) and Molina, M.D.C. et al. Journal of Pharmaceutical Sciences 90:1445-1455 (2001); Allison, S.D., et al. Biochemical et Biophysical Acta 1468:127-138 (2000)). In addition, Tf linked through PEG to a PEI-DNA polyplex retained some biological activity after freezing and thawing (Kursa, M. et al., Bioconjugate Chemistry 14:222-231 (2003)). It is important to note that this complex was not 89612.628
lyophilized, no indication of possible length of storage or condition given, and sugar (glucose), if included, was added after thawing. This polymer complex requires the Tf to be linked to the polymer through a PEG molecule. U.S. Patent No. 5,811,406 discloses polynucleotide complexes stabilized by adding a cryoprotectant compound and lyophilizing the resulting formulation. This reference does not disclose a liposome with a targeting ligand, let alone a lyophilized cell-targeting complex as described herein.

### Summary of the Invention

A method for preparing a stable complex comprising a ligand and a cationic liposome encapsulating a therapeutic or diagnostic agent or reporter gene comprises:
combining a complex comprising a ligand and a cationic liposome encapsulating a diagnostic or therapeutic agent or reporter gene with a solution comprising a stabilizing amount of sucrose as described in claim 1; and
lyophilizing the resultant solution of complex and sucrose to obtain a lyophilized preparation;
wherein, upon reconstitution, the preparation retains at least about 80% of its pre-lyophilization activity.

In a preferred embodiment, the preparation retains at least about 85% of its pre-lyophilization activity, and more preferably, at least about 90% of its pre-lyophilization activity.

### Brief Description of the Figures

Figure 1 shows the size (nm) of the freshly prepared and lyophilized complexes (Tf-LipA-Luc and TfRscFv-LipA-Luc) containing 5% dextrose or 5% sucrose.

Figure 2A and 2B shows the *in vitro* transfection efficiency, given as relative light units (RLU) per ug protein, of freshly prepared and lyophilized complexes (Tf-LipA-Luc and TfRscFv-LipA-Luc) containing 5% dextrose or 5% sucrose in DU145 human prostate cells.

Figure 3 shows the comparison of the *in vitro* transfection efficiency of freshly prepared and lyophilized
TfRscFv-LipA-Luc complex (RLU/ug protein) containing 5% or 10% sucrose in DU145 human prostate cancer cells.

Figure 4A and 4B, respectively, show DU145 prostate and PANCI pancreatic xenograft tumor targeting by lyophilized ligand-liposome plasmid DNA complexes.

Figure 5 demonstrates in a human prostate cancer (DU145) xenograft mouse model that the tumor targeting ability of laboratory prepared TfRscFv-LipA-p53 complex with 10% sucrose is maintained after lyophilization and storage at 2°-8°C for up to six months.

Figure 6 shown the batch to batch tumor targeting and transfection efficiency consistency of lyophilized TfRscFv-LipA-p53 complexes with 10% sucrose in a DU145 xenograft mouse model.

Figure 7 shows the tumor targeting and transfection efficiency of five different commercially prepared and lyophilized batches of TfRscFv-LipA-p53 with 10% sucrose in a DU145 xenograft mouse model.

Figure 8 shows the ,estimated percent of uncomplexed TfRscFv in various fresh and lyophilized TfRscFv-LipA-p53 complexes by non-denaturing gel electrophoresis.

Figure 9 shows the *in vitro* comparison of cell growth inhibition between lyophilized and freshly made TfRscFv-LipA-AS-HER-2 complexes in MDA-MB-435 breast cancer cells.

Figure 10 shows the *in vitro* comparison of PANC-1 chemosensitization by freshly prepared or lyophilized TfRscFv-LipA-AS HER-2 complexes.

Figure 11 shows the *in vitro* down modulation of HER-2 expression in MDA-MB-435 human breast cancer cells by TfRscFv-LipA-AS HER-2 with 10% sucrose after lyophilization and storage at 2°-8°C for up to six months.

### Detailed Description of the Invention

In accordance with the present invention, the stability of lyophilized complexes of a ligand and a liposome encapsulating a diagnostic or therapeutic agent can be increased by combining the complexes prior to lyophilization with an aqueous solution of a stabilizing amount of sucrose. The sucrose solution can be simply sucrose in water or a buffer can be included, such as PBS, HEPES, TRIS or TRIS/EDTA. Typically the sucrose solution is combined with the complex to a final concentration of about 1% to about 80% sucrose, typically 1% to about 50% sucrose, such as 1% to about 10%, 20%, 30% or 40% sucrose, preferably about 5% to 10% sucrose, and most preferably about 10% sucrose. The lyophilized preparation is stable within a range of from about 2-8°C to about -80°C for a period of at least 6 months without losing significant activity. Preferably the preparation is stable for a period of art least about 6-12 months. Upon reconstitution, the complexes retain at least about 80% of their pre-lyophilization activity, preferably at least about 85% of their pre-lyophilization activity and most preferably at least about 90 - 95% of their pre-lyophilization activity.

Previous reports have indicated that a mixture of lipid and DNA could be lyophilized in the presence of mono or disaccharides and maintain biological activity (Li, B., et al., Journal of Pharmaceutical Sciences 89:355-364 (2000) and Molina, M.D.C. et al. Journal of Pharmaceutical Sciences 90:1445-1455 (2001); Allison, S.D., et al. Biochemical et Biophysical Acta 1468:127-138 (2000)). It is unexpected, however, that a three component complex consisting of 1) a protein (e.g transferrin), including even a protein which is an antibody or antibody fragment (e.g., anti-transferrin receptor single chain antibody fragment, TfRscFv); 2)a liposome and 3) a therapeutic nucleic acid molecule (e.g. a plasmid DNA, an antisense oligonucleotides molecule or even an siRNA molecule) also could be lyophilized and retain both its size and biological activity after reconstitution.

The liposome complexes typically are administered intravenously. For intravenous injection, a 50% dextrose solution conventionally has been added to the ligand-liposome complexes to a final concentration of 5%. It now surprisingly has been found that by combining freshly prepared (i.e., a complex that is no more than about 1 to about 24 hours old) ligand-liposome complexes with a solution of sucrose, rather than dextrose, the activity and shelf life of the three component complexes (including those with an antigen targeting entity) following lyophilization and reconstitution can be significantly increased.

The three component complexes can simply be mixed with a sucrose solution prior to lyophilization. Typically the solution comprises about 50 to about 100% sucrose by weight, preferably about 50% by weight sucrose. Lyophilization can be in accordance with any conventional procedure that reduces the moisture content of the complex to less than about 1.3%. One preferred procedure comprises lyophilizing the complex-containing solution at -50°C to - 60°C, 20-50 millitorr, preferably 25 millitorr, for 12 to 60 hours, preferably 20-48 hours, then storing the lyophilized preparation between about 2-8°C and about -80°C. In another preferred procedure, vials containing the solution of complex are loaded into a commercial type lyophilizer at ambient temperature, then the temperature is ramped to -45°C ± 3°C over 1 hour and held at that temperature for three hours. The condenser then is chilled at -80°C or colder and the vacuum is set to 50 micron Hg. The shelf temperature is then ramped to -35° ± 3°C over 1 hour and once there held at this temperature for about 36-72 hours, preferably about 48 hours. The shelf temperature then is ramped to 20° ± 3°C over 4 hours and held at this temperature for about 6 to about 48 hours, preferably about 12 hours. At the end of this process the chamber pressure is restored to atmospheric with nitrogen (passed through an appropriate sterilizing microbial retentive filter) and the vials stoppered.

The lyophilized complexes can be reconstituted by the addition of sterile, endotoxin-free water equal to the volume of solution prior to lyophilization. The dried complexes dissolve rapidly with gentle rocking. No appreciable changes in size of the complex or zeta potential occurs due to the lyophilization or storage.

Suitable complexes which can be mixed with a sucrose solution, lyophilized and reconstituted are cell-targeting ligand/liposome/therapeutic, reporter or diagnostic molecule complexes that are capable of cell-targeted, systemic delivery of a variety of types of therapeutic or diagnostic molecules for use in treating or diagnosing diseases. The target cell preferably is a cancer cell, but can be a non-cancer cell as well. Preferred cancer target cells include prostate, pancreatic, breast, head and neck, ovarian, liver and brain cancers and melanoma. It is well known to one of ordinary skill in the art that most types of cancer cells, including, but not limited to, those listed above, overexpress the receptor for transferring and folate and that these receptors also rapidly recycle in cancer cells (Li, H., and Qian, Z.M., Medicinal research Reviews 2(3):225-250 (2000); Qian, Z.M., et al., Pharmacological Reviews 54(4):561-587 (2002); gosselin, M.A., and Lee, P.J., Biotechnology annual Reviews 8:103-131 (2002)).

Desirably, the therapeutic molecule is a gene, polynucleotide, such as plasmid DNA, DNA fragment, oligonucleotide, oligodeoxynucleotide, antisense oligonucleotide, chimeric RNA/DNA oligonucleotide, RNA, siRNA, ribozyme, viral particle, growth factor, cytokine, immunomodulating agent, or other protein, including proteins which when expressed present an antigen which stimulates or suppresses the immune system. Preferred therapeutic agents are nucleic acid molecules, preferably DNA or siRNA molecules. A preferred DNA molecule is one which encodes a gene such as a wild type p53 molecule, an Rb94 molecule, an Apoptin molecule, an EGFG molecule or an antisense molecule. A preferred HER-2 antisense oligonucleotide is against the HER-2 gene and has the sequence 5'-TCC ATG GTG CTC ACT-3'.
A preferred siRNA molecule is one which acts against HER-2 mRNA. Other preferred therapeutic molecules can be determined by one of ordinary skill in the art without undue experimentation.

As noted above, the target cell alternatively can be a non-cancer cell. Preferred non-cancer target cells include dendritic cells, endothelial cells of the brood vessels, lung cells, breast cells, bone marrow cells and liver cells. Undesirable, but benign, cells can be targeted, such as benign prostatic hyperplasia cells, overactive thyroid cells, lipoma cells, and cells relating to autoimmune diseases, such as B cells that produce antibodies involved in arthritis, lupus, myasthenia gravis, squamous metaplasia, dysplasia and the like.

Alternatively, the agent can be a diagnostic agent capable of detection in vivo following administration. Exemplary diagnostic agents include electron dense material, magnetic resonance imaging agents and radiopharmaceuticals. Radionuclides useful for imaging include radioisotopes of copper, gallium, indium, rhenium, and technetium, including isotopes ⁶⁴Cu, ⁶⁷Cu, ¹¹¹In, ^{99m}Tc, ⁶⁷Ga or ⁶⁸Ga. Imaging agents disclosed by Low et al. in U.S. Patent 5,688,488,
are useful in the liposomal complexes described herein.

The ligand can be any ligand the receptor for which is differentially expressed on the target cell. Examples include transferrin, folate, other vitamins, EGF, insulin, Heregulin, RGD peptides or other polypeptides reactive to integrin receptors, antibodies or their fragments. A preferred antibody fragment is a single chain Fv fragment of an antibody.

The antibody or antibody fragment is one which will bind to a receptor on the surface of the target cell, and preferably to a receptor that is differentially expressed on the target cell. One preferred antibody is an anti-TfR monoclonal antibody and a preferred antibody fragment is an scFv based on an anti-TfR monoclonal antibody. Another preferred antibody is an anti-HER-2 monoclonal antibody, and another preferred antibody fragment is an scFv based on an anti-HER-2 monoclonal antibody.

The ligand is mixed with the liposome at room temperature and at a ligand:liposome ratio in the range of about 1:0.001 to 1:500 (µg:nmole), preferably about 1:10 to about 1:50 (µg:nmole). The therapeutic agent is mixed with the cationic liposome at room temperature and at an agent:lipid ratio in the range of about 1:0.1 to about 1:50 (µg:nmole), preferably about 1:10 to about 1:24 (µg:nmole). In complexes, for example, in which the ligand is transferrin and the therapeutic agent is plasmid DNA, useful ratios of therapeutic agent to liposome to ligand typically are within the range of about 1 µg: 0.1-50 nmoles: 0.1 -100 µg, preferably 1 µg: 5-24 nmoles:6-36 µg, most preferably about 1 µg: 10 nmoles: 12.5 µg. If the ligand is TfRscFv, useful ratios of ligand to liposome typically are within the range of about 1:5 to 1:40 (µg:µg), preferably 1:30 (µg:µg), and the ratio of plasmid DNA to liposome typically is within the range of about 1:6 to 1:20 (µg:µg), preferably 1:14 (µg:µg). If the therapeutic agent is an oligonucleotide (ODN) rather than plasmid DNA, typical ratios of ligand, liposome and the ODN are 0.1 nmole to 36 nmole (ODN:liposome) and 0.1 µg to 100 µg (ligand:liposome), preferably 0.5 nmoles to 20 nmoles (ODN:liposome) and 0.5 µg to 50 µg (ligand:liposome), most preferably 1 nmole to 15 nmole (ODN:liposome) and 1 µg to 30 µg (ligand:liposome). If the therapeutic agent is an siRNA, useful ratios of the components can be 0.1 µg to 30 nmole (siRNA:liposome) and 0.1 µg to 100 µg (TfRscFv:liposome), preferably 1 µg to 7 nmole (siRNA:lipsosome) and 1 µg to 30 µg (TfRscFv:liposome).

A wide variety of cationic liposomes are useful in the preparation of the complexes. Published PCT application WO99/25320 incorporated herein by reference, describes the preparation of several cationic liposomes. Examples of desirable liposomes include those that comprise a mixture of dioleoyltrimethylammonium phosphate (DOTAP) and dioleoylphosphatidylethanolamine (DOPE) and/or cholesterol (chol), or a mixture of dimethyldioctadecylammonium bromide (DDAB) and DOPE and/or cholesterol. The ratio of the lipids can be varied to optimize the efficiency of uptake of the therapeutic molecule for the specific target cell type. The liposome can comprise a mixture of one or more cationic lipids and one or more neutral or helper lipids. A desirable ratio of cationic lipid(s) to neutral or helper lipid(s) is about 1:(0.5-3), preferably 1:(1-2) (molar ratio).

In one embodiment, the liposome used to form the complex is a sterically stabilized liposome. Sterically stabilized liposomes are liposomes into which a hydrophilic polymer, such as PEG, poly(2-ethylacrylic acid) or poly(n-isopropylacrylamide) (PNIPAM) have been integrated. Such modified liposomes can be particularly useful when complexed with therapeutic or diagnostic agents, as they typically are not cleared from the blood stream by the reticuloendothelial system as quickly as are comparable liposomes that have not been so modified. In a second embodiment, the liposome used to form the complex is also bound to a peptide composed of histidine and lysine (either branched or linear) where the peptide is at least about 10 amino acids in length, typically between about 10 and 1000 amino acids in length, and is composed of 5-100% histidine and 0-95% non-histidine amino acids; preferably at least 10% of the non-histidine amino acids are lysine. Most preferably the peptide is about thirty-one amino acids, approximately 20% of which are histidine and approximately 80% of which are non-histidine. Of these, at least 75% are lysine and at least one is a terminal cysteine. A preferred peptide has the structure 5'-K[K(H)-K-K-K]₅-K(H)-K-K-C-3' and can be covalently conjugated to the liposome through the terminal cysteine and a maleimide group in the liposome. In such complexes, the ratios of the components typically can be as follows: ligand to HK-liposome (µg: µg) of 1:5 to 1:40, preferably, 1:30 and DNA to HK-liposome (µg:nmole) of 1:1 to 1:20, preferably 1:14.

The complexes can be prepared by mixing the ligand-liposome and the therapeutic or diagnostic agent together, slowly inverting the resultant solution a number of time or stirring the solution at a speed where a vortex just forms in the solution for a period ranging from about 10 seconds to about 10 minutes, preferably 15 seconds to about 2 minutes.

The complexes can be administered in combination with another therapeutic agent, such as either a radiation or chemotherapeutic agent. The therapeutic agent, or a combination of therapeutic agents, can be administered before or subsequent to the administration of the complex, for example within about 12 hours to about 7 days. Chemotherapeutic agents include, but are not limited to, for example, doxorubicin, 5-fluorouracil (5FU), cisplatin (CDDP), docetaxel, gemcitabine, paclitaxel, vinblastine, etoposide (VP-16), camptothecia, actinomycin-D, mitoxantrone and mitomycin C. Radiation therapies include gamma radiation, X-rays, UV irradiation, microwaves, electronic emissions and the like.

The invention is further illustrated by the following examples which are provided for illustrative purposes and are not intended to be limiting.

### Examples

### Example 1

### Preparation of Fresh and Lyophilized Complexes with Carbohydrate and In Vitro Assessment of Activity and Size

Initial experiments were performed to test both the size and the *in vitro* transfection efficiency of the ligand-liposome nucleic acid complexes made with carbohydrate before and after lyophilization. Two separate ligands, Tf and TfRscFv, were tested. The complexes were made using the methodology described in U.S. Patent Application 09/601,444 and published U.S. Patent Applications 09/914,046 and 10/113,927 [See also, Xu, L., et al. Human Gene Therapy 10:2941-2952 (1999); Xu, L., et al., Human Gene Therapy 13:469-481 (2002); and Xu, L., et al., Molecular Cancer Therapeutics 1:337-346 (2002)]. In each complex, the liposome was a 1:1 ratio of DOTAP:DOPE, identified herein as Liposome A (LipA). The DNA used was a plasmid carrying a gene encoding the firefly luciferase gene. In all cases the carbohydrate solution was added as the last step in preparation of the complex.

A series of 8 complexes was made. Four contained Tf as the ligand (at a ratio of DNA:LipA:Tf of 1 µg:10 nmoles:12.5 µg); four contained TfRscFv as the ligand (at a ratio of DNA:LipA:TfrscFv of 1 µg:14 nmoles:0.34 µg). The solutions containing the ligand-liposome and the DNA were mixed together, slowly inverted 10 times, and the resultant solution was held at room temperature for 15 minutes prior to the addition of an aqueous solution of dextrose or sucrose in water to a final concentration of 5%. Each resultant admixture was inverted 10 times and then held at room temperature for 15 minutes prior to lyophilization or transfection.

The solutions to be lyophilized were lyophilized using a Virtis Benchtop 3L lyophilizer at 25 millitorr for 24 hours, at -55°C and then stored overnight at -80°C prior to reconstitution. After reconstitution with a volume of water equal to the volume of solution prior to lyophilization, the container holding each solution was slowly inverted 10 times and held at room temperature for 60 minutes. After this time the reconstituted complex could be kept at 2°-8°C for up to 24 hours. The size of the complexes before and after lyophilization were measured by dynamic laser light scattering using a Malvern Zetasizer 3000H.

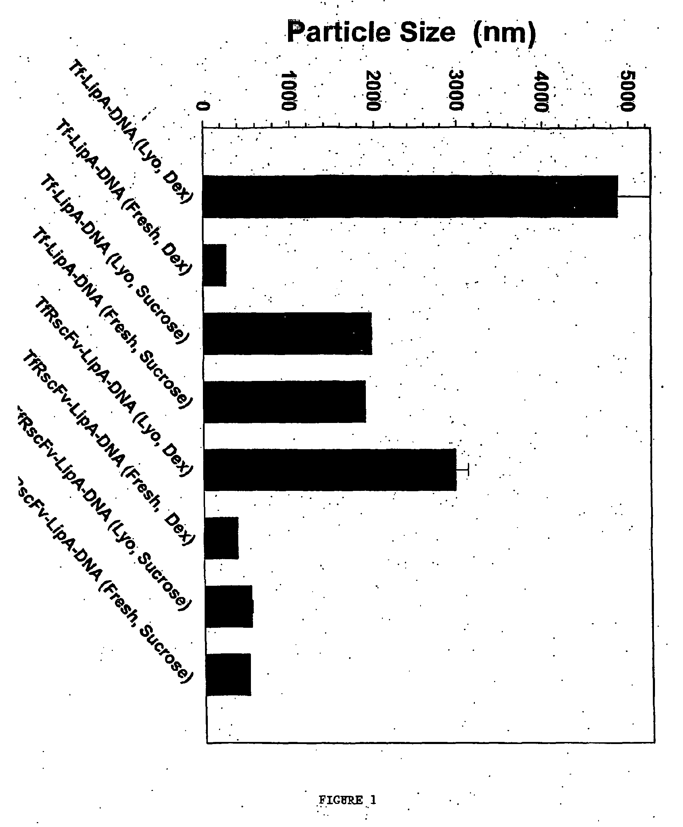

The results of the sizing (number average) are shown in Figure 1. When Tf was the ligand there was an approximate 10 fold increase in size after lyophilization in the presence of 5% dextrose. While the size of the fresh complex with 5% sucrose was slightly larger than that with 5% dextrose, there was essentially no change pre/post lyophilization in the presence of sucrose. A similar pattern was observed when TfRscFv was used as the ligand. Here also use of 5% sucrose gave much better post-lyophilization results.

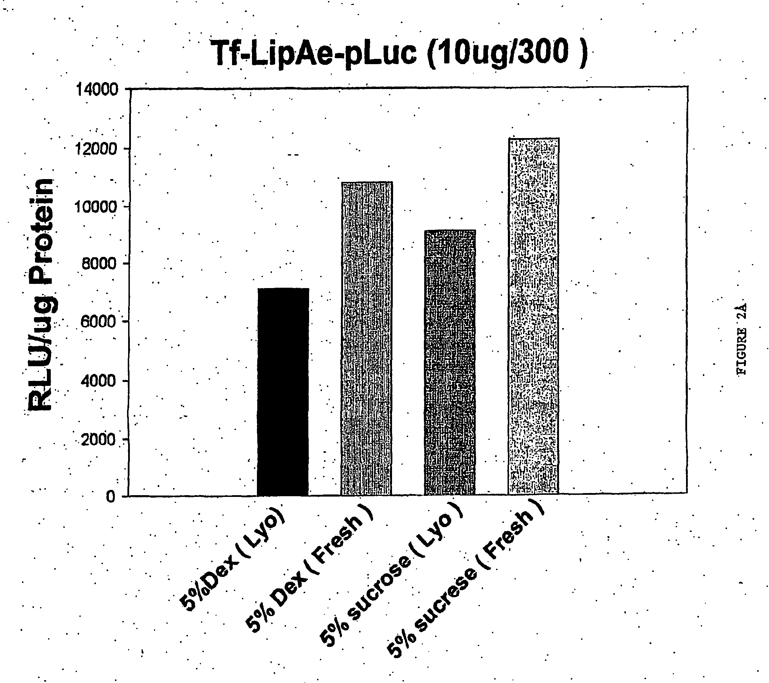

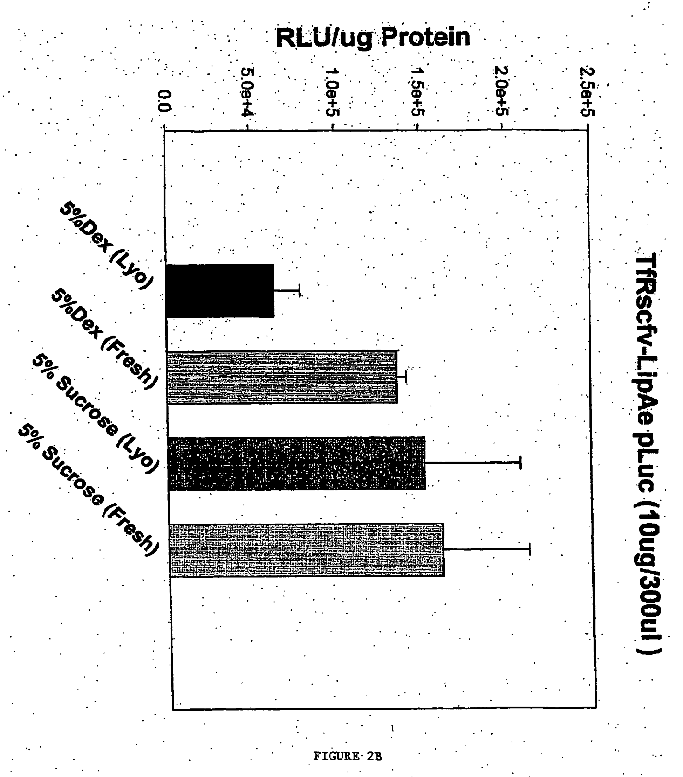

The fresh and lyophilized complexes also were assessed for their transfection efficiency in a human prostate tumor cell line DU145. The transfection efficiencies of the lyophilized complexes (with Tf and TfRscFv) upon reconstitution and corresponding freshly made solution of the same complex were compared. The results of the transfection efficiency pre- and post- lyophilization is shown in Figures 2A and 2B. When the ligand was Tf, the efficiency dropped after lyophilization to about 60% of that of the freshly prepared complex for the preparation containing 5% dextrose, whereas the lyophilized preparation containing 5% sucrose retained about 80% of its initial activity. The pattern was similar with the TfRscFv ligand. The lyophilized complex containing 5% dextrose dropped to about 50% of the fresh activity whereas about 90% of the activity was retained when 5% sucrose was used (Fig 2B). Thus, sucrose is a more efficient stabilizer than dextrose.

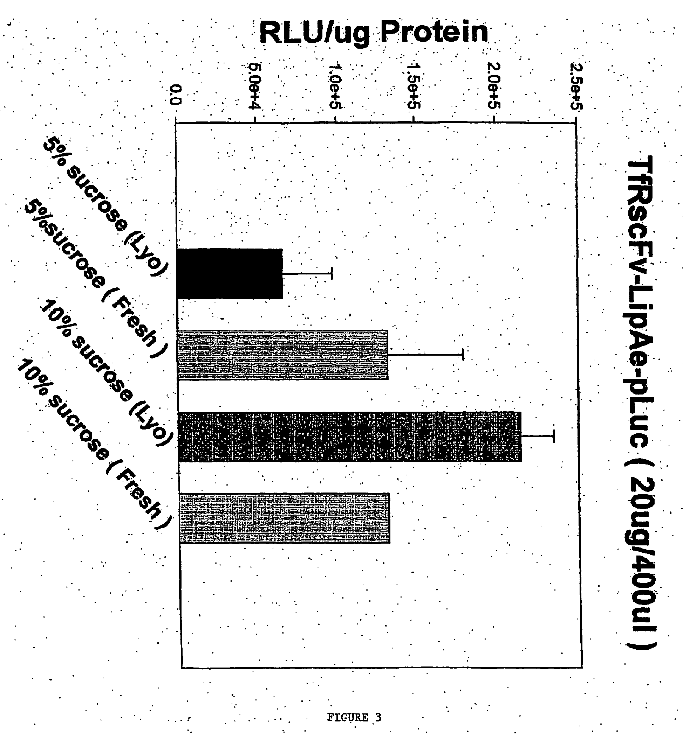

The sugar/complex ratio was further optimized to improve the stability and maintain particle size. Complexes with 5% and 10% sucrose were compared. The amount of plasmid DNA also was increased to 20ug, the amount customarily with used for a singe injection in the in vivo studies discussed below. After lyophilization as described above, the transfection efficiency of the complex containing 10% sucrose was -95% of that seen with the fresh complex prepared the conventional way with 5% dextrose solution. Figure 3 shows a comparison of transfection efficiency between complexes prepared with 5% and 10% sucrose. The *in vitro* transfection efficiency was best with the lyophilized complex containing 10% sucrose. This was found to be true independent of whether the protein or the antibody fragment was used as targeting ligand. The size of the complexes containing 10% sucrose also were assessed before and after lyophilization using the conditions given above. There was no significant difference between the sizes of the complexes made with 10% sucrose, either before and after lyophilization, as compared to the conventional freshly prepared complex made with 5% dextrose. Here also this was found to be the case independent of the targeting ligand.

Thus, the presence of 10% sucrose in a reconstituted liposome complex preparation resulted in higher maintenance of biological activity and size than that obtained with comparable reconstituted preparations containing either 5% dextrose or 5% sucrose.

### Example 2

### In Vivo Human Prostate Tumor Targeting by Lyophilized Complex After Storage at 2-8°C for One Week

*In vivo* tumor targeting of a liposome complex with 10% sucrose (freshly made or lyophilized and stored for 1 week at 2-8°C was tested using enhanced green fluorescence protein (EGFP) as the reporter gene in the complex. The complex was TfRscFv-Liposome A-pEGFP where liposome A is DOTAP:DOPE (1:1). The ratio of the three components was 0.3 µg:14 nmoles:1 µg (TfRscFv:Liposome:DNA). The complex was prepared and lyophilized as described above in Example 1. Post-lyophilization, the complex was stored refrigerated at 2-8°C for one week. The samples were reconstituted by the addition of endotoxin free water to a volume equal to that prior to lyophilization as described in Example 1.

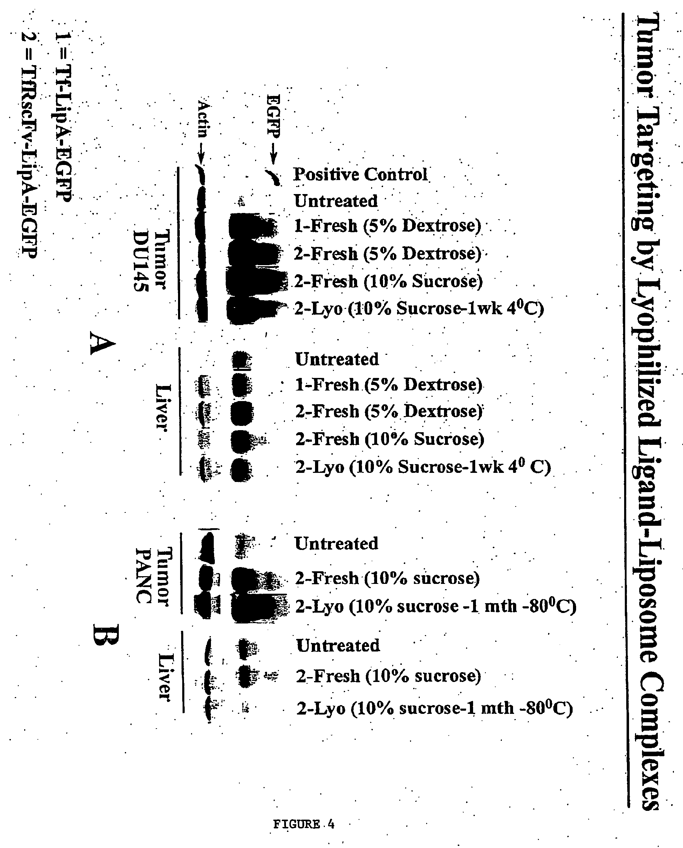

Mice bearing DU145 xenograft tumors of at least 50 mm³ were i.v. injected 3 times over 24 hours with various complexes (freshly made preparation with 5% dextrose; freshly made preparation with 10% sucrose and a reconstituted lyophilized preparation with 10% sucrose which prior to reconstitution had been held refrigerated for 1 week at 2-8°C all with TfRscFv as the targeting ligand). After 48 hours, the animals were sacrificed, tumor and liver excised, protein isolated and Western analysis performed using anti-EGFP Ab (COVANCE). As shown in Figure 4A, the lyophilized and reconstituted complex with 10% sucrose had a comparable level of gene expression compared to either of the freshly prepared complexes. More significantly, while a high level of exogenous gene expression was evident in the tumors, almost no EGFP was expressed in the livers of the mice, demonstrating the tumor specific nature of the complex, and that this tumor targeted specificity was maintained after lyophilization, storage at 2°-8°C for at least one week and reconstitution.

### Example 3

### In Vivo Human Pancreatic Tumor Targeting by Lyophilized complex After Storage at -80°C for One Month

The stability of the lyophilized complex also was tested after one month of storage at -80°C by targeting to pancreatic cancer xenograft tumors. The complex (the same complex and ratio as described above in Example 2) was prepared with 10% sucrose ad lyophilized as described in Example 1. Post-lyophilization the samples were stored at - 80°C for one month, then reconstituted with endotoxin free water as described in example 1.

As shown in Figure 4B, the tumors from mice i.v. injected 3 times over a 24 hour period (as in Example 2 above) showed an even higher level of EGFP gene expression than found after injection with the freshly prepared complex. Again, very little or no expression was seen in the liver.

### Example 4

### Long-Term Stability of the Lyophilized Complex Stored as 2-8°C as Assessed by Size and In vitro Transfection Efficiency

To increase the potential of our TfRscFv-liposome-DNA complex as a viable clinical therapeutic, a means of increasing its stability was developed, thus maintaining its tumor-targetability, and shelf-life. Some of our studies have indicated that the lyophilized complex with 10% sucrose as the excipient could be stored successfully at either - 20°C, -80°C or 2-8°C. For convenience for use in the clinic the preferred method of storage is 2-8°C. To determine the length of time the lyophilized complex can be stored at 2-8°C without loss of biological activity, the *in vitro* transfection efficiency of complex lyophilized and stored at 2-8°C for 1, 4 and 6 months was evaluated. The complex was TfRscFv-Liposome A-p53 where liposome A is DOTAP:DOPE (1:1). The ratio of the three components was 0.3 µg: 14 nmoles: 1 µg (TfRscFv: Liposome A: DNA) which is equivalent to 0.34 µg:10 µg:1 µg. 10% sucrose was used as the excipient. The DNA in the complex was a plasmid vector containing ∼1.7 Kb cDNA sequence coding for human wild-type p53. The complex was prepared, lyophilized and reconstituted at the appropriate time after storage at 2-8°C as described in Example 1. Size (number parameter) and Zeta Potential were determined using a Malvern 3000H Zetasizer. Functional activity was assessed using a luciferase co-transfection assay. Human prostate cancer DU145 cells were co-transfected with BP100 plasmid DNA and with the complexes. BP100 plasmid carries the luciferase gene under the control of a wtp53 inducible promoter. Thus, the level of functional p53 in the transfected cells is reflected by the level of luciferase activity. 24 hours after transfection, the cells were lysed and luciferase activity assayed, using the Promega Luciferase Reagent according to manufacturing protocol. As shown in Table 1, the luciferase activity, size and zeta potential of the complexes are consistent between the freshly prepared complex and complexes lyophilized and stored at 2-8°C for up to six months.

**Table 1**

| Comparison of Lyophilized Complex With Freshly Made Complex | | | |
|---|---|---|---|
| | Luciferase Activity (RLU/µg) | Size (nm) | Zeta Potential |
| UT | -- | -- | -- |
| BP100 | -- | -- | -- |
| Fresh-1 | 19,550 | -- | 25.3 |
| Fresh-2 | 19,825 | 453.7 (100%) | 8.0 |
| Lyo 1mo-1 | 15,420 | 450.8 (99.3%) | 28.9 |
| Lyo 1mo-2 | 13,879 | 463.3 (99.8%) | 33.2 |
| Lyo 4mo-1 | 20,055 | 462.5 (99.4%) | 29.1 |
| Lyo 4mo-2 | 18,413 | 554.2 (99.4%) | 27.5 |
| Lyo 4mo-3 | 22,058 | 548.5 (100%) | 29.4 |
| Lyo 6mo-1 | 14,507 | 550 (99.7%) | 23 |
| Lyo 6mo-2 | 13,301 | 414.5 (99.4%) | 28 |
| Lyo 6mo-3 | 15,028 | 386.4 (99.4%) | 25.6 |

### Example 5

### In Vivo Tumor Specific Targeting of the Systemically Administered Lyophilized Complex After Storage at 2-8°C

The fresh and lyophilized complexes, prepared, stored at 2-8°C for 1, 4, or 6 months, and then reconstituted for the studies described in Example 4 were also tested *in vivo* for their ability to reach and transfect human prostate xenograft tumors after systemic (i.v.) administration. Athymic nude mice bearing subcutaneous human prostate tumor cell line DU145 xenograft tumors of at least 100 mm³ were i.v. injected three times over 24 hours with complex (fresh or lyophilized and reconstituted) in an amount equivalent to 40 µg of DNA per injection in a final volume of 0.8 mL. At 48 hours after the last injection the animals are humanely euthanized, the organs removed, protein isolated and expression determined by Western Analysis as described by Xu, L. et al., Tumor Targeting 4:92-104 (1999). Other methods commonly known in the art alternatively could have been used. 80µg of total protein lysate was loaded/lane of a 12% SDS-polyacrylamide gel. After the gel was run, protein was transferred to nitrocellulose membrane and probed with an anti-p53 mouse monoclonal antibody (Oncogene Research Products).

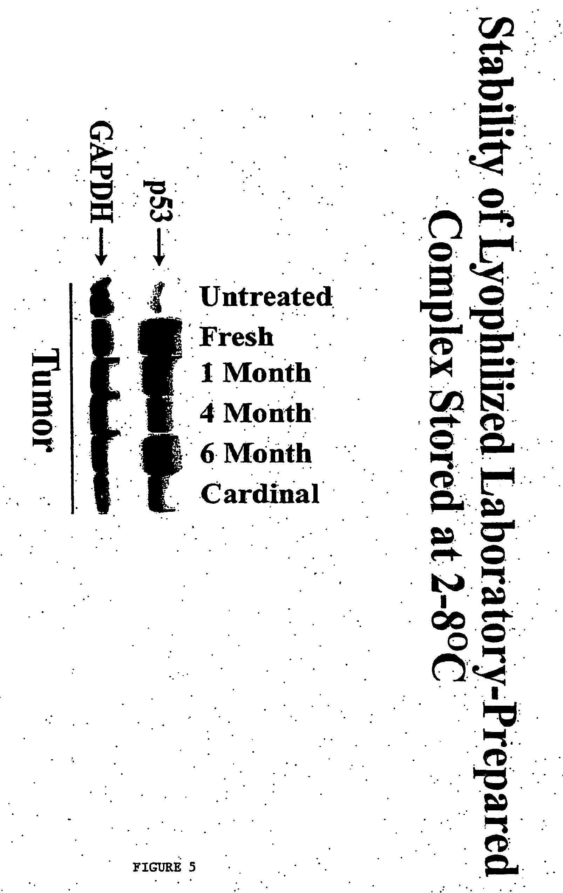

The results of the *in vivo* tumor targeting in mice are shown in Fig.5. The levels of p53 expression were similar between those in the tumor from animals receiving the freshly prepared complex and those.from animals receiving each of the lyophilized complexes even six months after lyophilization. p53 expression levels in all tumors were significantly higher than those in the liver, demonstrating that the tumor specificity after i.v. administration is maintained even after 6 months storage at 2-8°C.

### Example 6

### Batch to Batch Consistency and Stability After Lyophilization

It is important to establish that multiple batches of complex, prepared and lyophilized at different times on the same day and prepared and lyophilized on different days have similar sizes and levels of transfection efficiency. The complex TfRscFv-Liposome A-p53, where liposome A is DOTAP:DOPE (1:1) was prepared, lyophilized, stored and reconstituted as described in Example 1. The ratio of the components and the p53 DNA were as described In Example 4.

Functional expression of multiple TfRscFv-LipA-p53 complexes either freshly prepared or lyophilized was evaluated in prostate cancer DU145 cells. *In vitro* activity was assessed using the BP100 plasmid and the luciferase assay as described above in Example 4. On 5 different days at least two independent samples were prepared and lyophilized. After being stored at 2-8°C for 2 weeks, the samples were reconstituted as in Example 1 and tested *in vitro* and *in vivo. In vitro,* the luciferase activity (RLU/µg: Relative Light units per µg of protein in cell) was assayed, using the Promega Luciferase Reagent as described in the manufacture's protocol, and the zeta potential and the particle size (number parameter)of each batch were also measured on a Malvern 3000H Zetasizer. As shown in Table 2, by number parameter, size of the majority of the preparations falls into the 400-700 nm range and the zeta potentials are all in the positive range. Thus, different complexes made on different days have consistent behavior.

**Table 2**

| | | | | Sizing | | |
|---|---|---|---|---|---|---|
| Sample # | | | Luciferase Activity (RLU/µg Protein) | By Number (nm) | | Zeta Potential |
| ½/03 | 1 | | 17284 ± 1175 | 100% | 466 | 16 |
| ½/03 | 2 | | 21703 ± 2422 | 99% | 509 | 32 |
| 1/3/03 | | 1 | 16246 ± 1121 | 100% | 575 | 38 |
| 1/3/03 | | 2 | 19225 ± 1118 | 100% | 477 | 32 |
| 1/7/03 | | 1 | 17921 ± 1564 | 100% | 537 | 39 |
| 1/7/03 | | 2 | 17519 ± 3029 | 95% | 444 | 31 |
| 1/8/03 | | 1 | 21534 ± 3028 | 100% | 668 | 29 |
| 1/8/03 | | 2 | 21528 ± 3922 | 100% | 750 | 32 |
| 1/9/03 | | 1 | 18146 ± 1612 | 100% | 478 | 60 |
| 1/9/03 | | 2 | 12521 ± 172 | 91% | 645 | 38 |
| Fresh | | | 12806 | 66% | 147 | 38 |
| Fresh | | | 13173 | 100% | 258 | 40 |
| BP 100 | | | 131 | NA | | NA |
| UT | | | 103 | NA | | NA |

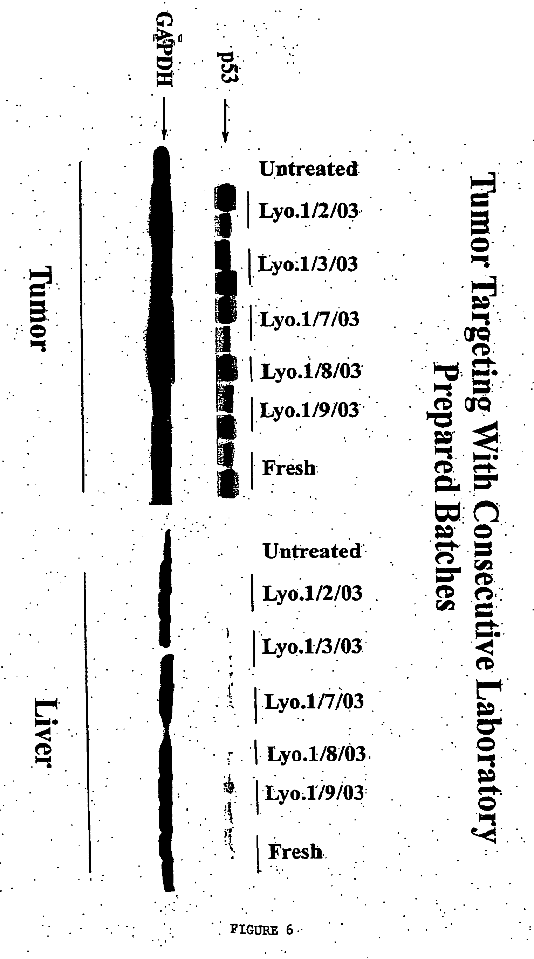

The samples also have been evaluated in vivo in DU145 xenograft bearing athymic nude mice as described in Example 5. To demonstrate that the ligand-liposome-DNA complex employing TfRscFv as the targeting entity maintains tumor specificity, Western analysis was employed (Figure 6). these independent batches of TfRscFv-LipA-p53 complex were tested in the DU145 human prostate subcutaneous xenograft mouse model. Nude mice carrying DU145 tumors of ∼50-100 mm³ were intravenously injected three times over a 24 hour period with the complexes (40µg DNA/injection). Twenty-four hours after the last injection the animals were sacrificed and the tumor and liver excised. Protein was isolated. 80 µg of total protein lysate were loaded/lane of a 12% SDS-polyacrylamide gel. After the gel was run, protein was transferred to nitrocellulose membrane and probed with an anti-p53 mouse monoclonal antibody (Oncogene Research Products). The membrane was subsequently probed for GAPDH levels to demonstrate equal loading. High p53 expression levels are evident in the tumor from the mice receiving the complexes containing each of the various batches (Fig.6). Similar level of p53 expression were observed in the tumors either with the freshly prepared or lyophilized TfRscFv complex. In contrast, very low p53 expression is observed in the mice not treated with the complex. The tumor specificity is demonstrated by the very low levels of expression in the liver. An all instances, there is a 5-10 fold difference in expression between the tumor and liver. Compared to the level of p53 in the untreated tumor, all preparations gave strong p53 signal in the tumors of treated mice but not in the corresponding livers in the same mice. Therefore, these studies indicate that lyophilization of the complete complex is feasible and may be able to overcome the problem of stability and shelf-life.

### Example 7

### Lyophilization by a Commercial Manufacturer: In Vitro and In Vivo Testing

For a lyophilized complex to be useful in treating human patients it is necessary to show that the process of complex preparation in the presence of 10% sucrose could be transferred and successfully performed on a large scale by a commercial manufacturing entity. The complexes were prepared by stirring, under contract and a confidentiality agreement, by Cardinal Health, Albuquerque, NM. The DNA solution was added to the TfRscFv:liposome solution while stirring at a speed where a vortex was just forming in the solution for 30 seconds to 1 minute. This solution was held at room temperature for 10 - 20 minutes, after which an aqueous solution of 50% sucrose was added with stirring as above for 30 seconds to 1 minute to a final concentration of 10% and held at room temperature for 10 - 20 minutes. The commercially prepared batches ranged in size from 50-1000 ml. The lyophilization protocol using a Hull lyophilizer at this commercial facility was as follows:
- 10 mL vials, each containing 5mL of complex with 10% sucrose, were loaded at ambient temperature.
- The shelf temperature was ramped to -45ºC over 1 hour. Once the shelf temperature reached -45 ± 3ºC, the product was held for 3 hours.
- At this point, the condenser was chilled to -55ºC or colder and the vacuum was set to 50 micron Hg.
- The shelf temperature was than ramped to -35ºC over 1 hour.
- Once the shelf temperature reached -35ºC, the product was held for 48 hours.
- The shelf temperature was ramped to 20ºC over 4 hours and the product was held for 12 hours.
- At the end of the cycle, the chamber pressure was restored to atmospheric with nitrogen, NF filtered through an appropriate sterilizing microbial retentive filter.
- The product was stoppered, labeled and stored at 2-8ºC.

Five different batches of the TfRscFv-LipA-p53 complex were prepared by the commercial entity. An example of the *in vitro* luciferase activity, size and zeta potential of representative commercially prepared batches are shown in Table 3. The size zeta potential and level of luciferase activity of the commercially prepared and lyophilized complexes was comparable to that of the complex freshly prepared in the laboratory.

**Table 3**

| Sample | Luciferase Activity (RLU/µg protein) | Size by Number (mm) | Zeta Potential |
|---|---|---|---|
| Fresh | 16.7 x 10⁴ | 413 | 35 |
| Commercial | 12.4 x 10⁴ | 416 | 29.8 |
| | 16.4 x 10⁴ | 412 | 30.9 |

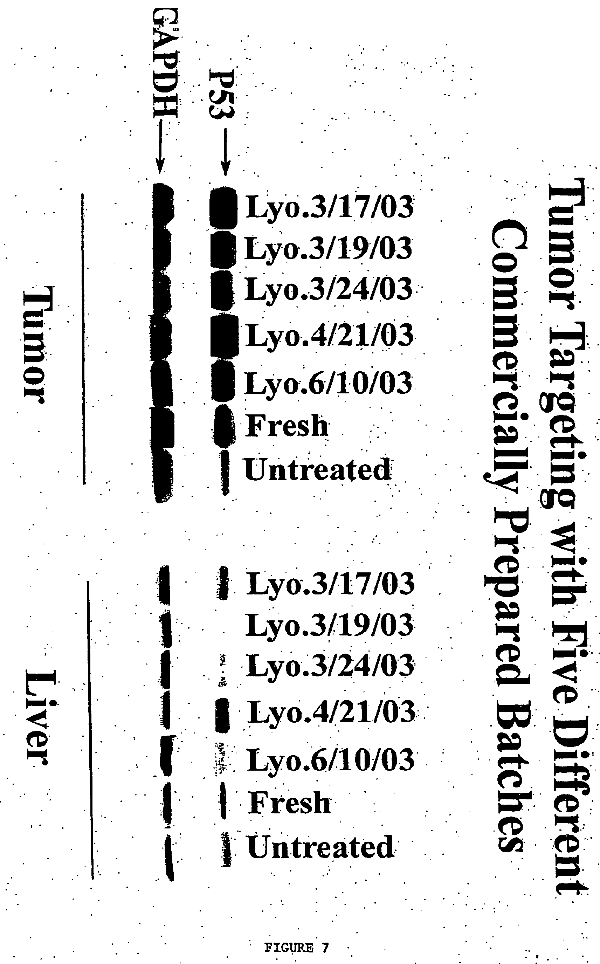

To compare the five batches, mice bearing DU145 xenografts were treated as described in Example 5 (at 40 µg DNA/injection in 0.8mL). Each mouse received three i.v. injections over 24 hours. Forty-eight hours after the last injection the animals were sacrificed and organs harvested. All five batches show high levels of p53 expression by Western Analysis that were comparable to that of the freshly prepared complex and significantly higher than that observed in either untreated tumor or liver (Fig.7). Therefore, this technology can be successfully transferred to commercial manufacturers.

### Example 8

### Consistency of Percent of Uncompleted TfRscFv Levels in the Lyophilized Complex

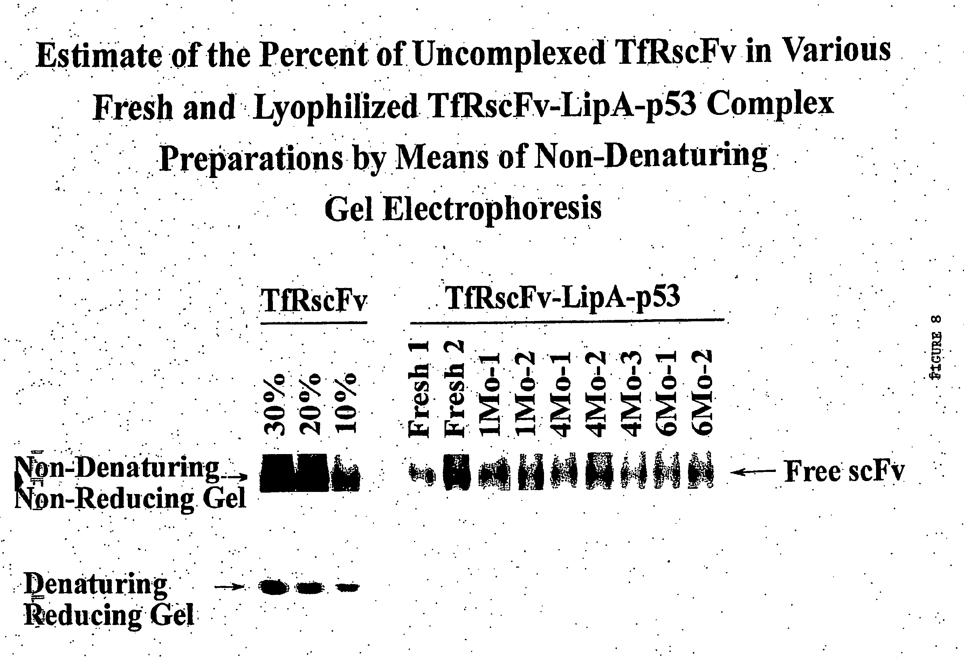

To further assess the stability of the lyophilized complex, the amount of uncomplexed ligand was determined after storage at 2-8°C for up to six months. To evaluate the amount of uncomplexed TfRscFv present in the TfRscFv-LipA-p53 complex, 4%-20% gradient non-denaturing and non-reducing polyacrylamide gel electrophoresis followed by Western analysis was employed using methods commonly known to one skilled in the art (Fig.8). A polyclonal rabbit antibody against the TfRscFv protein was used as the first antibody (produced by Animal Pharm, Healdsburg, CA) and a HRP-labeled mouse anti-rabbit monoclonal antibody (Sigma) as the second antibody. Freshly made or lyophilized complexes containing 134 ng of TfRscFv in each were prepared and lyophilized as described in Example 1. The lyophilized samples were stored at 2-8°C for 1, 4, or 6 months, after which they were reconstituted as in Example 1. Once complexed to liposomes, the TfRscFv protein will not be able to enter the PAGE gel. Therefore, only the uncomplexed free TfRscFv will be detected. It is difficult, under non-denaturing and non-reducing conditions, to accurately determine the amount of the free TfRscFv monomer. Thus, uncomplexed samples containing 13.4 ng (10% of that in each of the complexes), 26.8ng (20%) or 40.2ng (30%) of the single agent TfRscFv were also run in the same gel as concentration standards for a rough estimate of the amount of the uncomplexed TfRscFv in each of the test complexes.

The results indicated that approximately 10% or less of the TfRscFv initially put into the complex is present as free TfRscFv in the various fresh or lyophilized preparations of TfRscFv-LipA-p53 complex even after storage at 2-8°C for six months. These data suggest that the amount of free, uncomplexed TfRscFv is quite consistent in all preparations, and that this level does not change after lyophilization and storage at 2-8°C for at least six months.

### Example 9

### Lyophilization of Ligand-Liposome-Nucleic Acid Complex Containing an Antisense HER-2 Oligonucleotide

The above studies used plasmid DNA in the complex. Since plasmid DNA and oligonucleotides are not always interchangeable, and can have different chemistries, experiments also were carried out to demonstrate that the lyophilization procedure could be applied to a ligand-liposome complex containing an oligonucleotides (ODN). The ODN used was a 15 mer phosphorothioated sequence specific antisense HER-2 ODN complementary to the initiation codon region of the HER-2 gene (AS HER-2) with the sequence 5'-TCC ATG GTG CTC ACT-3'. Using MDA-MB-453 human breast cancer cell line as the assay system, cell killing by the TfRscFv-lipA-AS HER-2 complex was evaluated after lyophilization with different sugars at increasing ODN concentrations. The complexes were prepared as described in Example 1 and were composed of TfRscFv, Liposome A (DOTAP:DOPE at 1:1) and the ODN at a ratio of 1 nmole to 15 nmole (ODN:liposome) and 1 µg to 30 µg (TfRscFv:liposome).

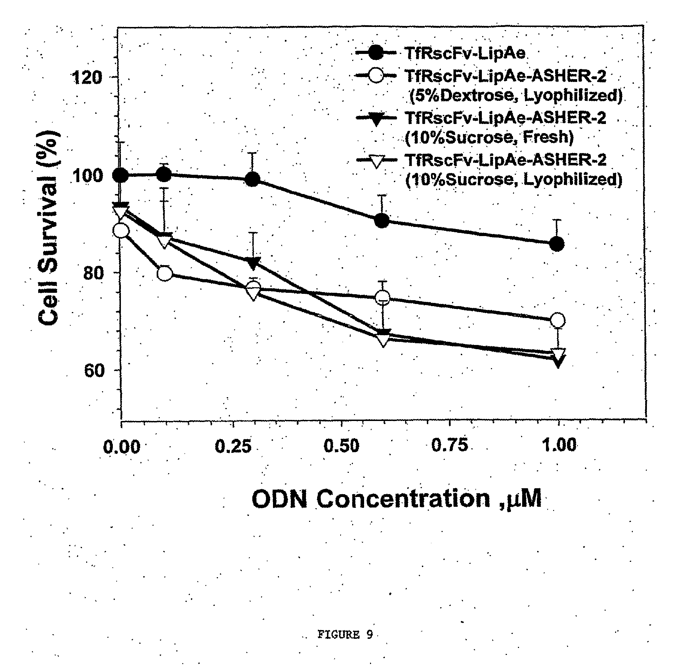

The complexes to be lyophilized were prepared to contain either 5% dextrose or 10% sucrose and compared to freshly prepared comparable complex preparations comprising 10% sucrose. The complexes were lyophilized as described in Example 1, stored overnight at 2-8°C and reconstituted in endotoxins-free water as described in Example 1. 5 x 103 MDA-MB-453 cells were seeded/well of a 96-well plate. 24 hours later the cells were transfected with either the freshly prepared or lyophilized and reconstituted complexes. The cell viability XTT-based cytotoxicity assay (XTT=3'-[1-phenyl-Amino-Carbonyl)-3,4-[tetrazolium]-bis(4-methoxy-6-nitro)benzene sulfonate) was performed in triplicate 48 hours post-transfection. As shown in Figure 9, at AS-HER-2 ODN concentrations above 0.25 µM, the lyophilized and reconstituted complex containing 10% sucrose had the greatest effect on cell killing. At the higher concentrations of ODN, both the fresh and reconstituted 10% sucrose-containing complexes were far superior to that with 5% dextrose and lyophilization had no adverse effect on the cell-killing ability of the HER-2 antisense ODN contained in the complex.

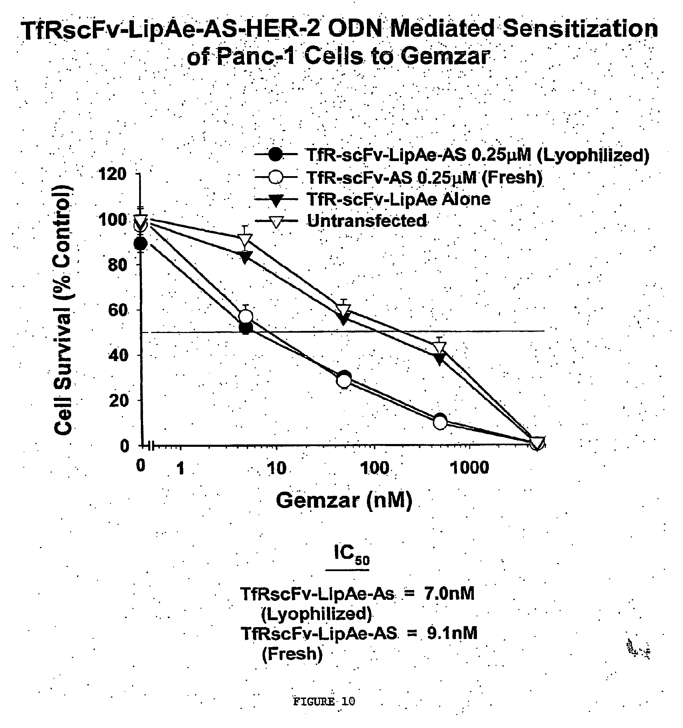

To confirm that lyophilization and reconstitution in the presence of 10% sucrose was not detrimental to the efficacy of the complex, an XTT assay assessing the level of chemosensitization to Gemzar in human pancreatic cancer (PANC) 1 cells was preformed, comparing freshly prepared complexes against comparable complexes that had been lyophilized and reconstituted as described in Example 1. The ratios of the components in the complex were 1 nmole : 15 nmole (ODN:liposome) and 1 µg : 30 µg (TfRscFv:liposome). 4 x 10³ PANC-1 cells were seeded/well of a 96 well plate and transfected 24 hours later with TfRscFv-LipA-AS HER-2 (0.25µM ODN) complex that was either freshly prepared or had been mixed with sucrose to provide 10% sucrose and lyophilized, stored refrigerated overnight at 2-8°C and reconstituted. The chemotherapeutic drug Gemzar was added 24 hours later. The cell viability XTT-based assay was performed in triplicate 72 hours after drug addition. The results are illustrated in Figure 10. As shown, the survival curves of the samples were virtually identical. In addition, the IC₅₀ (the concentration of drug killing 50% of the cells) values of the complexes are the same, if not lower, than previously determined using a freshly prepared complex with 5% dextrose. The preparation and storage method of this invention thus also is amenable to use with any antisense oligonucleotides since the target gene is irrelevant to the process.

These studies indicate that lyophilization of the complete complex is feasible and that previous difficulties with stability and shelf life when using ODN as therapeutic molecules can be overcome.

### Example 10

### Maintenance of Size, Zeta Potential and Efficacy After Lyophilization of Complex Carrying AS ODN and Storage for Six Months.

The size, zeta potential and transfection activity of the ligand-liposome-nucleic acid complexes containing AS HER-2 ODN and prepared with 10% sucrose were examined.before and after lyophilization. The size of the complex was found to be essentially the same before and after lyophilization and storage at -20°C for up to six months. For example: Pre-lyophilization, the values for size (nm) by intensity, volume and number average for the fresh and six month lyophilized complexes prepared as described in Example 9 were 410 (I), 454 (V) and 368 (N) *vs* 339 (I), 427 (V) and 397 (N), respectively. In addition, another oligonucleotide that does not affect HER-2 levels (SC-ODN_ (5'-CTA GCC ATG CTT GTC-3') was also complexed at the same ratio, lyophilized, stored for up to six months at -20°C and reconstituted as in Example 9. Here also lyophilization and storage had no significant effect on size or zeta potential of the complex. Thus, any ODN can be complexed and lyophilized.

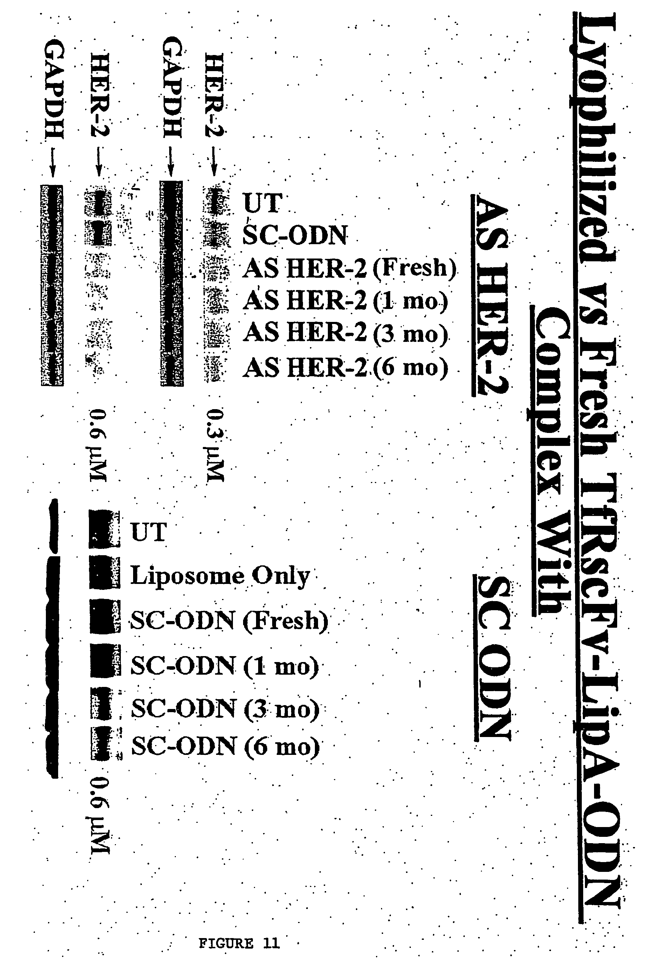

The zeta potentials were -43.8 (fresh) and -47.7 (lyophilized) after six months storage. The transfection efficiency of the lyophilized complex with 10% sucrose was measured by assessing the ability of the TfRscFv-lip A-AS HER-2 to down modulate HER-2 expression *in vitro*. After preparation, lyophilization (as in Example 4), and storage at -20°C for up to six months, the complex AS HER -2 ODN at two different concentrations (0.3 or 0.6µM) or SC-ODN at 0.6µM, were used to transfect human breast cancer cell line MDA-MS-435 cells. Freshly prepared complexes carrying AS HER-2 or SC-ODN were used as controls. The SC-ODN had no effect either before or after Lyophilization. However, there was an AS HER-2 ODN dose dependent down-modulation of HER-2 expression by both freshly prepared and lyophilized complexes (Figure 11) even after six months storage at - 20°C. Since the SC-ODN had no effect, the down-modulation observed was not a result of any general cytotoxicity due to lyophilization of the complex.

### Example 11

### Maintenance of Size and Zeta Potential of Complex Carrying siRNA After Lyophilization

The stability of a complex of TfRscFv, Liposome A and siRNA with 10% sucrose after lyophilization was assessed by measuring the size of the complex and the zeta potential before and after lyophilization. The complex was composed of TfRscFv, Liposome A (DOTAP:DOPE at 1:1 mole ratio) and siRNA at 33.3µg. Total volume of complex was 500 µL. The ratio of the components was 1 µg to 7 nmole (siRNA:liposome) and 1 µg to 30 µg (TfRscFv:liposome). Sucrose was added to the complex to a final concentration of 10%. The complex was prepared and lyophilized as described in Example 1. After lyophilization the complex was reconstituted as described in Example 1 and size and zeta potential were measured using a Malvern Zetasizer 3000H. The results are shown in Table 4.

**Table 4**

| Sample | Intensity | Size (nm) Volume | Number | Zeta Potential |
|---|---|---|---|---|
| Fresh | 416 | 437 | 31.9 (99%) | 5.0 |
| | | | 363 (1%) | |
| Lyophilized | 261 | 373 | 115 (94%) | 5.4 |
| | | | 392 (6%) | |

Therefore, after Lyophilization there was no significant change in size or zeta potential. If anything, the size by intensity and volume are even smaller after lyophilization, making the complex more efficient for *in vivo* use.

### Example 12

### Maintenance of Size Complex Made with Peptide-liposome After Lyophilization

To further demonstrate the general nature of this invention a complex also was prepared that contained a modified liposome. The liposome used to form the complex was bound to a peptide. The peptide comprised histidine and lysine and was a branched peptide 31 amino acids in length and was composed of a combination of histidine and non-histidine amino acids with the structure 5'-K[K(H)-K-K-K]₅-K(H)-K-K-C-3'). The liposome in this study was comprised of DOTAP:DOPE (1:1). The HK peptide was covalently conjugated to the liposome through the terminal cysteine and a maleimide group in the liposome. The complex consisted of TfRscFv-HK-liposome-DNA where the ratios of the components were as follows: TfRscFv to HK-liposome (µg: µg) of 1 µg:30 µg and DNA to HK-liposome (µg:nmole) of 1 µg: 14 nmole. The DNA used was p53 (see Example 4) at 18 µg DNA for 300 µL of total volume of complex. 10% sucrose was included in the final complex. The complex was prepared and lyophilized as described in Example 1. Post-lyophilization the complex was stored at 2-8°C for 3 days and then reconstituted as described in Example 1. The size of the complex before lyophilization and after three days storage at 2-8°C was measured on a Malvern Zetasizer 3000H. Prior to lyophilization the size (number average) was 601 nm. After storage and reconstitution it was 588. Thus, once again lyophilization of the complex using 10% sucrose did not result in any significant change in the size of the complex even with the inclusion of the HK peptide.

### Example 13

### In Vitro and In Vivo Assessment of Complex Carrying a Different Therapeutic Gene (RB94) After Lyophilization and Storage at 2°-8°C

In addition to the Luciferase gene, a gene coding for the enhanced green fluorescence protein, and the p53 gene, a liposome complex carrying other plasmid DNA can be lyophilized and retain size and biological activity. To further demonstrate, this complex was also prepared carrying another therapeutic gene, the tumor suppressor gene RB94. The complex was TfRscFv-liposome A-RB94 where liposome A is DOTAP:DOPE (1:1). The ratio of the three components (TfRscFV:Liposome:DNA) were 0.34 µg : 10 µg: 1µg. The complex also contained 30 µg of RB94 plasmid DNA in a total volume of 0.5mL, with 10% sucrose. The complex was prepared as described in Example 1 and lyophilized using the method described in Example 1 for the size and zeta potential studies, or prepared as in Example 7 by Cardinal Health using for use the *in vitro* and *in vivo* targeting studies.

### Size and Zeta Potential

The size and zeta potential of complex prepared as in Example 1 and lyophilized, stored at 2-8°C for four days and reconstituted as in Example 1 was compared before and after lyophilization and storage using a Malvern Zetasizer 3000H. Prior to lyophilization the size (nm) was intensity and 283 (Intensity) and 392 (Volume), while afterward it was found to be 303 (Intensity) and 347 (Volume). Thus, there was no significant change in size after lyophilization and storage for four days at 2°-8°C when 10% sucrose was included. Similarly the zeta potential showed no major difference, both being in the +20 to +30 range [19 (pre) and 30.7 (post)].

### In Vitro and In Vivo Targeting

The ability of the complex to specifically target tumor cells and efficiently transfect them after lyophilization and storage at 2-8°C for an extended period of time was also tested in cell culture using human prostate cell line DU145 and human bladder carcinoma cell line HTB-9. Both cell lines were transfected *in vitro* using either freshly prepared complex or complex that had been prepared and lyophilized by the commercial contractor (Example 7) and stored at 2°-8°C for approximately 4 months prior to reconstitution as in Example 1. The level of RB94 protein expression in the cells was determined by Western Analysis using standard protocols known to one skilled in the art. There was no significant difference in either human tumor cell line between the amount of protein detected after transfection with freshly prepared or lyophilized complex.

Mice carrying human bladder carcinoma HTB-9 xenograft tumors were injected systemically (i.v. via the tail vein) with the freshly prepared complex or complex that had been prepared with 10% sucrose by stirring as in Example 1, lyophilized (Example 7), and stored at 2°-8°C for almost 5 months prior to reconstitution in Example 1. The mice received a total of three i.v. injections over 24 hours (40 µg of DNA in 0.67 mL per injection). Approximately 48 hours after the last injection the animals were humanely sacrificed, the tumors and liver excised, and protein obtained and analyzed by Western Blot using an anti-RB94 monoclonal antibody (QED Biosciences, Inc) by means of a common procedure as described by Xu, L., et al., tumor Targeting 4:92-104 (1999). As with the *in vitro* studies there was no significant difference in the level of RB94 protein evident in the tumors from the animals receiving the fresh or the lyophilized complexes. If anything, the expression was even higher in the tumors from the mice injected with the lyophilized complex. Moreover, there was virtually no expression in the livers in either group demonstrating that the tumor targeting ability of the complex was maintained after lyophilization in the presence of 10% sucrose and storage at 2-8°C for at least 5 months.

## Claims

1. A method for preparing a stable cell-targeting complex comprising a ligand and a cationic liposome encapsulating a therapeutic agent, reporter gene or diagnostic agent which comprises:
providing a complex comprising a ligand and a cationic liposome encapsulating a diagnostic agent, reporter gene or therapeutic agent;
combining said liposome complex with an aqueous solution consisting essentially of a stabilizing amount of sucrose in water or in a buffer to a final concentration of about 1% to about 80% sucrose; and
lyophilizing said solution of liposome complex and sucrose to obtain a lyophilized preparation;
wherein, upon reconstitution, said preparation retains at least about 80% of its pre-lyophilization activity.

2. The method of claim 1, wherein said solution consists essentially of sucrose in a PBS buffer, in a HEPES buffer, in a TRIS buffer or in a TRIS/EDTA buffer.

3. The method of claim 1 or claim 2, wherein said preparation retains at least about 85% or at least about 90% or at least about 95% of its pre-lyophilization activity upon reconstitution.

4. The method of any one of claims 1 to 3, wherein said complex is combined with a sucrose solution to a final concentration of about 1% to about 80% or about 1% to about 50% or about 1% to about 20% or about 5% to about 10% or about 10% sucrose.

5. The method of any one of claims 1 to 4, wherein said ligand comprises a ligand the receptor for which is differentially expressed on a target cell.

6. The method of any one of claims 1 to 5, wherein said ligand is a protein.

7. The method of claim 5, wherein said ligand comprises transferrin, folate, an antibody or an antibody fragment.

8. The method of claim 7, wherein said ligand comprises an anti-TfR antibody.

9. The method of claim 7, wherein said ligand comprises a single chain Fv fragment of an antibody.

10. The method of claim 9, wherein said antibody fragment comprises an scFv based on an anti-TfR antibody.

11. The method of any one of claims 1 to 10, wherein said liposome comprises at least one cationic lipid and at least one neutral or helper lipid.

12. The method of claim 11, wherein said cationic lipid comprises dioleoyltrimethylammonium phosphate (DOTAP) or dimethyldioctadecylammonium bromide (DDAB) and said neutral or helper lipid comprises dioleoylphosphatidylethanolamine (DOPE) or cholesterol (chol).

13. The method of claim 11, wherein said liposome comprises a mixture of DOTAP and DOPE.

14. The method of any one of claims 1 to 13, wherein the liposome is bound to a peptide of at least about 10 amino acids, wherein said peptide is composed of about 5-100% histidine and 0-95% non-histidine residues.

15. The method of claim 14, wherein at least 10% of said non-histidine residues of said peptide are lysine residues.

16. The method of claim 15, wherein said peptide has the structure 5'-K[K(H)-K-K-K]₅-K(H)-K-K-C-3'.

17. The method of any one of claims 1 to 16, wherein said therapeutic agent comprises a gene, plasmid DNA, oligonucleotide, oligodeoxynucleotide, antisense oligonucleotide or siRNA.

18. The method of any one of claims 1 to 16, wherein said diagnostic agent comprises electron dense material, magnetic resonance imaging agents or radiopharmaceuticals.

19. A lyophilized preparation comprising a complex of a ligand and a cationic liposome encapsulating a therapeutic agent, or reporter gene or diagnostic agent which is stable at a temperature in the range of about -80°C to 8°C for at least about six months while retaining at least about 80% activity, said preparation comprising said complex and about 1% to about 80% sucrose to increase the stability of said complex.

20. The lyophilized preparation of claim 19, which comprises about 1% to about 50% or about 1% to about 20% or about 5% to about 10% or about 10% sucrose.

21. The lyophilized preparation of claim 19, which upon reconstitution retains at least about 80% or about 85% or about 90% or about 95% of its pre-lyophilization activity.

22. The lyophilized preparation of any one of claims 19 to 21, wherein said ligand, said therapeutic agent said diagnostic agent, and said liposome are as defined in any one of claims 5 to 18.

## Patentansprüche

1. Verfahren zum Herstellen eines stabilen Zelltargetingkomplexes mit einem Liganden und einem kationischen Liposom, das ein Therapeutikum, Reportergen oder Diagnostikmittel einkapselt, welches Verfahren folgende Schritte aufweist:
Bereitstellen eines Komplexes mit einem Liganden und einem kationischen Liposom, das ein Therapeutikum, Reportergen oder Diagnostikmittel einkapselt;
Kombinieren des Liposom-Komplexes mit einer wässrigen Lösung, die im Wesentlichen aus einer stabilisierenden Menge an Saccharose in Wasser oder in einem Puffer mit bzw. bis zu einer abschließenden Konzentration von ungefähr 1 % bis ungefähr 80% Saccharose besteht; und
Lyophilisieren der Lösung aus Liposom-Komplex und Saccharose, um eine lyophilisierte Zubereitung zu erhalten;
wobei nach Rekonstitution die Zubereitung mindestens etwa 80 % ihrer Prälyophilisierungs-Aktivität beibehält.

2. Verfahren nach Anspruch 1, wobei die Lösung im Wesentlichen aus Saccharose in einem PBS-Puffer, in einem HEPES-Puffer oder in einem TRIS-Puffer besteht.

3. Verfahren nach Anspruch 1 oder 2, wobei die Zubereitung nach Rekonstitution mindestens etwa 85 % oder mindestens etwa 90 % oder mindestens etwa 95 % ihrer Prälyophilisierungs-Aktivität beibehält.

4. Verfahren nach einem der Ansprüche 1 bis 3, wobei der Komplex mit einer Saccharose-Lösung mit bzw. zu einer abschließenden Konzentration von ungefähr 1 % bis etwa 80 % oder etwa 1 % bis etwa 50 % oder etwa 1 % bis etwa 20 % oder etwa 5 % bis etwa 10 % oder etwa 10 % Saccharose kombiniert wird.

5. Verfahren nach einem der Ansprüche 1 bis 4, wobei der Ligand einen Liganden aufweist, für welchen der Rezeptor differentiell auf einer Targetzelle dargestellt bzw. exprimiert wird.

6. Verfahren nach einem der Ansprüche 1 bis 5, wobei der Ligand ein Protein ist.

7. Verfahren nach Anspruch 5, wobei der Ligand Transferrin, Folat, ein Antikörper oder ein Antikörperfragment aufweist.

8. Verfahren nach Anspruch 7, wobei der Ligand ein Anti-TfR Antikörper aufweist.

9. Verfahren nach Anspruch 7, wobei der Ligand ein Einfachketten-Fv-Fragment eines Antikörpers aufweist.

10. Verfahren nach Anspruch 9, wobei das Antikörperfragment ein scFv basierend bzw. basiert auf einem Anti-TfR Antikörper aufweist.

11. Verfahren nach einem der Ansprüche 1 bis 10, wobei das Liposomen mindestens ein kationisches Lipid und mindestens ein neutrales oder Helferlipid enthält.

12. Verfahren nach Anspruch 11, wobei das kationische Lipid die Dioleoyltrimethylammonium-Phosphat (DOTAP) oder Dimethyldioctadecylammonium-Bromid (DDAB) und das neutrale oder Helferlipid Dioleoylphosphatidylethanolamin (DOPE) oder Cholesterol (chol) aufweist.

13. Verfahren nach Anspruch 11, wobei das Liposom eine Mischung aus DOTAP und DOPE aufweist.

14. Verfahren nach einem der Ansprüche 1 bis 13, wobei das Liposomen an ein Peptid aus mindestens etwa 10 Aminosäuren gebunden ist, wobei das Peptid aus etwa 5 % - 100 % Histidin und 0 % - 95% Nichthistidinresten bzw. - rückständen zusammengesetzt ist.

15. Verfahren nach Anspruch 14, wobei mindestens 10 % des Nichthistidin-Restes des Peptides Lysin-Reste sind.

16. Verfahren nach Anspruch 15, wobei das Peptid die Struktur 5'K[K(H)-K-K-K] ₅-K(H)-K-K-C-3' aufweist.

17. Verfahren nach einem der Ansprüche 1 bis 16, wobei das Therapeutikum ein Gen, Plasmid DNA, Oligonukleotid, Oligodeoxynukleotid, Antisense-Oligonukleotid oder siRNA aufweist.

18. Verfahren nach einem der Ansprüche 1 bis 16, wobei das Diagnostikmittel elektronendichtes Material, Magnetresonanztomographie-Mittel oder Radiopharmazeutika aufweist.

19. Lyophilisierte Zubereitung mit einen Komplex aus einem Liganden und einem kationischen Liposom, das ein Therapeutikum, oder Reportergen oder Diagnostikmittel einkapselt, welches stabil in einem Temperaturbereich von etwa -80 ºC bis 8 ºC für mindestens etwa 6 Monate ist während es mindestens etwa 80 % der Aktivität beibehält, wobei die Zubereitung den Komplex und etwa 1 % bis 80 % Saccharose, um die Stabilität des Komplexes zu erhöhen, aufweist.

20. Lyophilisierte Zubereitung nach Anspruch 19, welche etwa 1 % bis etwa 50 % oder etwa 1 % bis etwa 20 % oder etwa 5 % bis etwa 10 % oder etwa 10 % Saccharose aufweist.

21. Lyophilisierende Zubereitung nach Anspruch 19, welche nach Rekonstitution mindestens etwa 80 % oder etwa 85 % oder etwa 90 % oder etwa 95 % ihrer Prälyophilisierungs-Aktivität beibehält.

22. Lyophilisierte Zubereitung nach einem der Ansprüche 19 bis 21, wobei der Ligand, das Therapeutikum, das Diagnostikmittel und das Liposom nach einem der Ansprüche 5 bis 18 definiert sind.

## Revendications

1. Procédé pour préparer un complexe de ciblage de cellules stable comportant un ligand et un liposome cationique encapsulant un agent thérapeutique, un gène rapporteur ou un agent de diagnostic, lequel procédé comporte les étapes consistant à :
fournir un complexe comportant un ligand et un liposome cationique encapsulant un agent de diagnostic, un gène rapporteur ou un agent thérapeutique ;
combiner ledit complexe de liposome avec une solution aqueuse essentiellement constituée d'une quantité de stabilisation de saccharose dans l'eau ou dans un tampon jusqu'à une concentration finale d'environ 1 % à environ 80 % de saccharose ; et
lyophiliser ladite solution de complexe de liposome et de saccharose afin d'obtenir une préparation lyophilisée ;
dans lequel, lors d'une reconstitution, ladite préparation conserve au moins environ 80 % de son activité de pré-lyophilisation.

2. Procédé selon la revendication 1, dans lequel ladite solution est essentiellement constituée de saccharose dans un tampon PBS, dans un tampon HEPES, dans un tampon TRIS ou dans un tampon TRIS/EDTA.

3. Procédé selon la revendication 1 ou la revendication 2, dans lequel ladite préparation conserve au moins environ 85 % ou au moins environ 90 % ou au moins environ 95 % de son activité de pré-lyophilisation lors d'une reconstitution.

4. Procédé selon l'une quelconque des revendications 1 à 3, dans lequel ledit complexe est combiné avec une solution de saccharose jusqu'à une concentration finale d'environ 1 % à environ 80 % ou d'environ 1 % à environ 50 % ou d'environ 1 % à environ 20 % ou d'environ 5 % jusqu'à environ 10 % ou d'environ 10 % de saccharose.

5. Procédé selon l'une quelconque des revendications 1 à 4, dans lequel ledit ligand comporte un ligand dont le récepteur est exprimé différentiellement sur une cellule cible.

6. Procédé selon l'une quelconque des revendications 1 à 5, dans lequel ledit ligand est une protéine.

7. Procédé selon la revendication 5, dans lequel ledit ligand comporte une transferrine, un folate, un anticorps ou un fragment d'anticorps.

8. Procédé selon la revendication 7, dans lequel ledit ligand comporte un anticorps anti-TfR.

9. Procédé selon la revendication 7, dans lequel ledit ligand comporte un fragment Fv à chaîne unique d'un anticorps.

10. Procédé selon la revendication 9, dans lequel ledit fragment d'anticorps comporte un scFv basé sur un anticorps anti-Tfr.

11. Procédé selon l'une quelconque des revendications 1 à 10, dans lequel ledit liposome comporte au moins un lipide cationique et au moins un lipide neutre ou auxiliaire.

12. Procédé selon la revendication 11, dans lequel ledit lipide cationique comporte du phosphate de dioléoyltriméthylammonium (DOTAP) ou du bromure de diméthyldioctadécylammonium (DDAB), et ledit lipide neutre ou auxiliaire comporte de la dioléoylphosphatidyléthanolamine (DOPE) ou du cholestérol (chol).

13. Procédé selon la revendication 11, dans lequel ledit liposome comporte un mélange de DOTAP et de DOPE.

14. Procédé selon l'une quelconque des revendications 1 à 13, dans lequel le liposome est lié à un peptide d'au moins environ 10 acides aminés, dans lequel ledit peptide est composé d'environ 5 à 100 % d'histidine et de 0 à 95 % de résidus non-histidine.

15. Procédé selon la revendication 14, dans lequel au moins 10 % desdits résidus non-histidine dudit peptide sont des résidus de lysine.

16. Procédé selon la revendication 15, dans lequel ledit peptide a la structure 5'-K[K(H)-K-K-K]₅-K(H)-K-K-C-3'.

17. Procédé selon l'une quelconque des revendications 1 à 16, dans lequel ledit agent thérapeutique comporte un gène, un ADN de plasmide, un oligonucléotide, un oligodésoxynucléotide, un oligonucléotide antisens ou un siARN.

18. Procédé selon l'une quelconque des revendications 1 à 16, dans lequel ledit agent de diagnostic comporte une substance dense en électrons, des agents d'imagerie par résonance magnétique ou des produits pharmaco-radioactifs.

19. Préparation lyophilisée comportant un complexe constitué d'un ligand et d'un liposome cationique encapsulant un agent thérapeutique ou un gène rapporteur ou un agent de diagnostic qui est stable à une température dans la plage d'environ - 80 °C à 8 °C pendant au moins environ six mois tout en conservant au moins 80 % d'activité, ladite préparation comportant ledit complexe et environ 1 % à environ 80 % de saccharose afin d'augmenter la stabilité dudit complexe.

20. Préparation lyophilisée selon la revendication 19, laquelle comporte environ 1 % à environ 50 % ou environ 1 % à environ 20 % ou environ 5 % à environ 10 % ou environ 10 % de saccharose.

21. Préparation lyophilisée selon la revendication 19 qui, lors d'une reconstitution, conserve au moins 80 % ou environ 85 % ou environ 90 % ou environ 95 % de son activité de pré-lyophilisation.

22. Préparation lyophilisée selon l'une quelconque des revendications 19 à 21, dans laquelle ledit ligand, ledit agent thérapeutique, ledit agent de diagnostic et ledit liposome sont comme défini dans l'une quelconque des revendications 5 à 18.
